(19) Europäisches Patentamt
European Patent Office
Office européen des brevets

(11) Publication number: **0 184 630**
**A2**

(12) # EUROPEAN PATENT APPLICATION

(21) Application number: 85112873.6

(22) Date of filing: 11.10.85

(51) Int. Cl.[4]: G01N 33/94, //G01N33/533,G01N33/58,G01-N33/70

(30) Priority: 25.10.84 US 664894

(43) Date of publication of application: 18.06.86 Bulletin 86/25

(84) Designated Contracting States: AT BE CH DE FR GB IT LI LU NL SE

(71) Applicant: ABBOTT LABORATORIES
14th Street and Sheridan Road North St
North Chicago Illinois 60064(US)

(72) Inventor: Bennett, Larry
84 W. Belvidere Road
Grayslake, IL 60030(US)
Inventor: Wang, Philip P.
608 Dawes Street
Libertyville, IL 60048(US)
Inventor: Dodge, Robert H.
15683 W. Idlewood Lane
Libertyville, IL 60048(US)

(74) Representative: Modiano, Guido et al
MODIANO, JOSIF, PISANTY & STAUB Modiano & Associati Via Meravigli, 16
I-20123 Milan(IT)

(54) **Fluorescence polarization assay, reagents, and method of making reagents, for determination of digitoxin.**

(57) The present invention is directed to an improved fluorescent polarization assay for digitoxin, and to improved reagents for use in the assay. The reagent is formed as the reaction product of 3-amino-3-deoxydigitoxigenin and 5- or 6-carboxyfluorescein, under conditions for forming amide linkages, to yield a tracer. The tracer forms a complex with antiserum specific to digitoxin, in proportion to the relative concentration of the tracer with respect to the concentration of the digitoxin present in the sample. It has been found that excellent results are obtained when such a reagent is used for measuring concentrations of digitoxin.

EP 0 184 630 A2

# FLUORESCENCE POLARIZATION ASSAY, REAGENTS, AND METHOD OF MAKING REAGENTS, FOR DETERMINATION OF DIGITOXIN

## BACKGROUND OF THE INVENTION

### 1. Technical Field

This invention related to a method and reagents for determining the amount of digitoxin in fluids, especially biological fluids such as serum or plasma, and to a method for making the reagents. Specifically, the invention relates to a fluorescence immunoassay procedure and to tracers employed as reagents in such a procedure.

### 2. Background Art

Digitoxin is a cardiac glycoside used to treat congestive heart failure. Although digitoxin is highly effective in increasing the force of myocardial contractions, dosage levels are critical since the therapeutic concentrations are very close to toxic levels. Since individuals may vary in their response, it is essential to carefully monitor the therapy by measuring the serum or plasma levels of the drug.

Typically, competitive binding immunoassays are used for measuring ligands in a test sample. (For the purposes of this disclosure, a "ligand" is a substance of biological interest to be quantitatively determined by a competitive binding immunoassay technique.) The ligands compete with a labeled reagent, or "ligand analog" or "tracer," for a limited number of receptor binding sites on antibodies specific to the ligand and ligand analog. The concentration of ligand in the sample determines the amount of ligand analog which binds to the antibody: the amount of ligand analog that will bind is inversely proportional to the concentration of ligand in the sample, because the ligand and the ligand analog each bind to the antibody in proportion to their respective concentrations.

In the past, patient serum/plasma digitoxin levels were measured by heterogeneous radioimmunoassays utilizing radioactive iodine (125-I) on the ligand analog. However, such assays were not without drawbacks. Typically, the radioactive assay kits had a short shelf life and posed handling and disposal problems due to the radioactivity. Additionally, the heterogeneous procedure required that the unbound radioactive tracer (ligand analog) be separated from the bound tracer before the reading could be taken. Yet another drawback was the relatively long incubation time (30-60 minutes) required by the radioimmunoassay procedures.

Fluorescence polarization provides a quantitative means for measuring the amount of tracer-antibody conjugate produced in a competitive binding immunoassay. Fluorescence polarization techniques are based on the principle that a fluorescent labeled compound, when excited by plane polarized light, will emit fluorescence having a degree of polarization inversely related to its rate of rotation. Accordingly, when a tracer-antibody conjugate having a fluorescent label is excited with plane polarized light, the emitted light remains highly polarized because the fluorophore is constrained from rotating between the time that light is absorbed and emitted. In contrast, when an unbound tracer is excited by plane polarized light, its rotation is much faster than the corresponding tracer-antibody conjugate and the molecules are more randomly oriented. As a result, the light emitted from the unbound tracer molecules is depolarized. Such fluorescence polarization techniques have been applied in U.S. Patent No. 4,420,568 to Wang et al., which is directed to the use of a triazinylaminofluorescein moiety as the fluorophore. The present invention offers an advance in the art beyond the Wang patent, in that highly sensitive tracers, a method for making the tracers, and an assay using the tracers are provided specifically for the determination of digitoxin. The assay conducted in accordance with the present invention is particularly accurate, as will be explained below.

## SUMMARY OF THE INVENTION

This invention is directed to tracers for use in fluorescent polarization assays for digitoxin, to methods for conducting such assays, and to methods of making the tracers.

A first aspect of the invention relates to the discovery of unique tracers having a novel structure. According to the first aspect of the present invention, there is provided N-(3'-(3'-deoxydigitoxigenin)-x-carboxyfluorescein-x-amide, wherein x is 5 or 6, i.e., a compound of the formula:

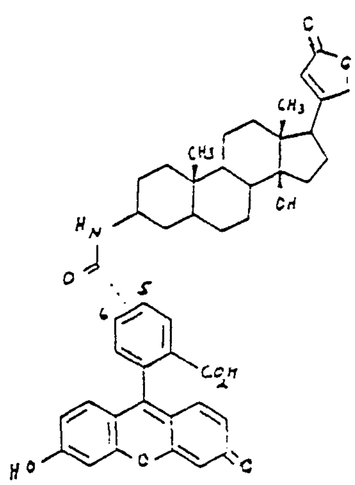

(Formula A)

The compound of Formula A can also be described as the reaction product of 3-amino-3-deoxydigitoxigenin with 5- or 6-carboxyfluoroscein. The dotted line indicates that the 3-amino-3-deoxydigitoxigenin moiety occupies the 5 or 6 position on the fluorescein moiety. The compound of Formula A is useful as a reagent for quantitatively determining the presence of digitoxin in blood serum in a homogeneous mixture of said reagent.

According to a second aspect of the invention, there is provided a synthesis method, i.e., a method for making the compound of Formula A by reacting 3-amino-3-deoxydigitoxigenin with 5- or 6-carboxyfluorescein. In a presently preferred embodiment, the reaction product is obtained under conditions for forming amide linkages, such as by preparing a solution of 5- or 6-carboxyfluorescein, contacting this solution with 1,3-dicyclohexylcarbodiimide (N,N-dicyclohexylcarbodiimide) and N-hydroxysuccinimide, and then reacting the 5- or 6-carboxyfluorescein mixture with a solution of 3-amino-3-deoxydigitoxigenin. In a most preferred embodiment, 5-carboxyfluorescein is the starting material of choice.

A third aspect of the invention relates to an analytical method, i.e., a method using as a reagent the compound of Formula A, of conducting an assay. According to the third aspect of the invention, an improved fluorescence polarization assay is provided, comprising the steps of contacting a sample with a digitoxin antiserum and a fluorescent-labeled digitoxigenin derivative capable of producing a detectable fluorescence polarization response to the presence of digitoxin antiserum in a homogeneous solution, passing plane polarized light through the homogeneous solution, and measuring the fluorescence polarization response therefrom.

Further objects and attendant advantages will be best understood from a reading of the following detailed description taken together with the drawings and the Examples.

## BRIEF DESCRIPTION OF THE DRAWINGS

Figure 1 shows the structure of digitoxin, the molecule whose concentration is measured by the analytical method of the invention.

Figure 2 shows the structure of the 5-carboxyfluorescein molecule, a preferred reactant for synthesizing the compound of Fig. 4.

Figure 3 shows the structure of the 3-amino-3-deoxydigitoxigenin molecule, another preferred reactant for synthesizing the compound of Fig. 4.

Figure 4 shows the structure of a preferred tracer of the present invention, N-(3'-(3'-deoxydigitoxigenin))-5-carboxyfluorescein-5-amide.

Figure 5 shows the structure of the 6-carboxyfluorescein molecule, an alternate to the reactant of Fig. 2.

Figure 6 shows the structure of a tracer of the present invention, N-(3'-(3'-deoxydigitoxigenin))-6-carboxyfluorescein-6-amide, an alternate tracer to that of Fig. 4.

Figure 7 shows a shorthand method of illustrating both the alternative structures of Figures 3 and 6.

Figure 8 corresponds to Table I, illustrates the structures to which reference is made in Table I.

## DETAILED DESCRIPTION OF THE PREFERRED EMBODIMENTS

The present invention is directed to reagents for use as tracers in an improved fluorescence polari zation immunoassay; to synthetic methods, for making the reagents; and to an analytical method, using the reagents, for quantitatively detecting the presence of digitoxin in fluids, such as human body fluids including blood serum, plasma, spinal fluid, and the like.

Specifically, it has been found in accordance with the invention that the reaction product of 3-amino-3-deoxydigitoxigenin and 5- or 6-carboxyfluorescein produces a digitoxin analog (tracer). This tracer is highly effective in fluorescence polarization assays for digitoxin. These assays can be carried out in a homogeneous solution, i.e., in a solution comprising both bound and unbound tracers, without the need for separating the two.

With respect to the method for making the tracers, the reaction products are coupled under conditions normally used to form amide linkages. It is preferred that active ester procedures be used, as these are most effective for forming the desired amide linkage in this particular situation. The reaction can be carried out under temperature conditions from about 0°C to about 100°C. (most preferably between about 0°C and about 80°C.), and the reaction time can vary between about one-half hour to about 144 hours. The presently preferred methods for making the tracers are set forth in detail in the Examples.

The tracers of the present invention generally exist in an equilibrium between their acid and ionized states, and in the ionized state are effective in the method of the present invention. Therefore, the present invention comprises the tracers in either the acid or ionized state, and for convenience, the tracers of the present invention are structurally represented herein in their acid form. When the tracers of the present invention are present in their ionized state, the tracers exist in the form of biologically accept able salts. As used herein, the term "biologically acceptable salts" refers to salts such as sodium, potassium, ammonium and the like which will enable the tracers of the present invention to exist in their ionized state when employed in the method of the present invention. Generally, the tracers of the present invention exist in solution as salts, with the specific salt resulting from the buffer employed. For example, in the presence of a sodium phosphate buffer, the tracers of the present invention will generally exist in their ionized state as a sodium salt.

A tracer which is not complexed to an antibody is free to rotate in less than the time required for absorption and re-emission of fluorescent light. As a result, the re-emitted light is relatively randomly oriented so that the fluorescence polarization of a tracer not complexed to an antibody is low, approaching zero. Upon complexing with a specific antibody, the tracer-antibody complex thus formed assumes the rotation of the antibody molecule which is slower than that of the relatively small tracer molecule, thereby increasing the polarization observed. Therefore, when a ligand competes with the tracer for antibody sites, the observed polarization of fluorescence of the tracer-antibody complex becomes a value somewhere between that of the tracer and tracer-antibody complex. If a sample contains a high concentration of the ligand, the observed polarization value is

closer to that of the free ligand, i.e., low. If the test sample contains a low concentration of the ligand, the polarization value is closer to that of the bound ligand, i.e., high. By sequentially exciting the reaction mixture of an immunoassay with vertically and then horizontally polarized light and analyzing only the vertical component of the emitted light, the polarization of fluorescence in the reaction mixture may be accurately determined. The precise relationship between polarization and concentration of the ligand to be determined is established by measuring the polarization values of calibrators with known concentrations. The concentration of the ligand can be extrapolated from a standard curve prepared in this manner.

The particular tracers formed in accordance with this invention, N-(3'-(3'-deoxydigitoxigenin))-5-carboxyfluorescein-5-amide and N-(3'-(3'-deoxydigitoxigenin))-6-carboxyfluorescein-5-amide, have been found to produce surprisingly good results in fluorescence polarization assays, with the former tracer being preferred over the latter.

It is believed that the effectiveness of the tracers in the assay is due to a combination of two factors. First, because the relatively small hydrolysis product derivative of digitoxin (i.e., 3-amino-3-deoxydigitoxigenin) is used in combination with a fairly small fluorophore moiety (i.e., 5- or 6-carboxyfluorescein), the liquid and fluorophere moieties are held closely together so that undesirable rotation between absorption and emission of polarized light when complexed with the antiserum, is strictly limited. Second, and most surprisingly, the particular tracers of the present invention display outstanding binding and displacement properties when complexed to digitoxin antiserum. The excellent binding is surprising because one of ordinary skill in the art would not expect the tracers to be well recognized by the antiserum in view of the substantial differences between the structure of the tracers and that of the digitoxin molecule. The latter comprises three sugar groups, all of which are absent in the tracer. The tracer comprises aromatic instead of the saturated rings of the sugars, and lacks the hydroxyl groups of the sugars in the digitoxin molecule. The excellent binding is especially surpris ing since the tracer and the digitoxin molecules are both relatively small, such that one of ordinary skill in the art would expect the antiserum binding to be highly selective and sensitive to any change in the digitoxin structure. Despite the fact that the relatively small digitoxin and tracer molecules have significantly different structures, the binding properties of the tracers of the present invention are outstanding, as will be more fully explained below.

The results can be quantified in terms of net millipolarization units, span (in millipolorization units) and intensity. The measurement of millipolarization units indicates the maximum polarization when a maximum amount of the tracer is bound to the antibody in the absence of any digitoxin. The higher the net millipolarization units, the better the binding of the tracer to the antibody. The span is an indication of the difference between the net millipolarization and the minimum amount of tracer bound to the antibody. A larger span provides for a better numerical analysis of data. The intensity is a measure of the strength of the signal above background. Thus, a higher intensity will give a more accurate measurement. The intensity is determined at about 2 nanomolar (i.e., approximately 1.8-2.2 nanomolar) concentration of tracer as the sum of the vertically polarized intensity plus twice the horizontally polarized intensity.

Table I shows that the results of the tracers of the present invention, in terms of net millipolarization units, span and intensity, are far superior to the results obtained when any of the other listed fluorophore moieties were used in conjunction with digitoxigenin. Additionally, attempts to use fluorescein derivatives in conjunction with unhydrolysed digitoxin molecules were unsuccessful. As noted above, it is believed that the advantages of the tracers of the present invention derive from the proximity of the digitoxigenin moiety and the fluorescein moiety, since when larger molecules bind to the antibody receptor sites, their polarization retention is relatively poor. However, the surprising aspect of the application of this theory is that one would not expect that the tracers of the present invention would be well recognized by the digitoxin specific antiserum, because of the substantial differences in structure between the relatively small digitoxin and tracer molecules. Thus, it should be understood that this theoretical hypothesis to explain the surprisingly good results obtained in accordance with the present invention should not be construed as limiting, but is intended simply as a plausible explanation of the success thereof.

TABLE I

TABLE I

| Structure | Fluorophore and linking* moiety | Net mP | Span (mP) | Intensity |
|---|---|---|---|---|
| I | 5-carboxyfluorescein (with amide linkage) | 208 | 119.48 | approx. 3000 |
| II | 6-carboxyfluorescein (with amide linkage) | 200 | 100 | approx. 1800 |
| III | 4-aminobenzoyl 5-carboxyfluorescein | 114.29 | 38 | 2400 |
| IV | piperazinyl 6-carboxyfluorescein urethane | 68 | 35 | 3500 |
| V | piperazine DTAF (isomer I) | 15.35 | <10 | 2400 |
| VI | piperazine DTAF (isomer II) | 50.27 | 0 | 2200 |
| VII | DTAF (isomer I) | 76.33 | 50 | 2200 |
| VIII | DTAF (isomer II) | 20 | <10 | 2800 |

| Structure | Fluorophore and linking* moiety | Net mP | Span (mP) | Intensity |
|---|---|---|---|---|
| IX | succinate fluorescein-amine (isomer I) | 12 | <10 | 3700 |
| X | 4-aminobutyryl-5-carboxy-fluorescein | 22 | 0 | 2300 |
| XI | hydroxylamine O-acetic acid fluoresceinamine (isomer I) | 11 | <10 | 1900 |
| XII | succinate fluoresceinamine (isomer I) | 22 | <10 | 2200 |
| XIII | phosgene fluoresceinamine (isomer II) | 64.69<br>58 | 50<br>68 | 2500<br>2400 |
| XIV | chlorosulfonyl isocyanate fluoresceinamine (isomer I) | 53.11<br>66.84 | 53<br>38 | 2400<br>3100 |
| XV | 5-carboxyfluorescein (without amide linkage-with ester linkage) | 3.84 | 0 | 2700 |
| XVI | tridigitoxose 5-carboxyfluorescein | 0 | 0 | 3900 |

* Linkage is to the 3 position of 3-deoxydigitoxigenin in structures I through XVI. Structure XVI is digitoxin with 5-carboxyfluorescein attached to the terminal sugar.

In accordance with the method of conducting the assay of the present invention, a sample containing digitoxin, or suspected of containing digitoxin, is intermixed with a biologically acceptable salt of a tracer, such as the tracers illustrated in Fig. 4 or Fig. 6, and an antibody specific for digitoxin and tracer. The tracer is a carboxyfluorescein digitoxigenin derivative. The digitoxin and the tracer compete for limited antibody sites resulting in the formation of digitoxin-antibody and tracer-antibody complexes. By maintaining constant the concentration of tracer and antibody, the ratio of digitoxin-antibody complex to tracer-antibody complex that is formed is directly proportional to the amount of digitoxin present in the sample. Therefore, upon exciting the mixture with plane polarized light and measuring the polarization of the fluorescence emitted by a tracer and a tracer-antibody complex, one is able to quantitatively determine the amount of digitoxin in the sample.

The pH at which the method of the present invention is practiced must be sufficient to allow the digitoxigenin tracers to exist in their ionized state. The pH may range from about 3 to 12, more usually in the range of from about 5 to 10, most preferably from about 6 to 9. Various buffers may be used to achieve and maintain the pH during the assay procedure. Representative buffers include borate, phosphate, carbonate, tris, barbital and the like. The particular

buffer employed is not critical to the present invention, but the tris and phosphate buffers are preferred. The cation portion of the buffer will generally determine the cation portion of the tracer salt in solution.

The reagents for the fluorescence polarization assay of the present invention comprise antibody specific for digitoxin, and either of the tracers N-(3'-(3'-deoxydigitoxigenin))-5-carboxyfluorescein-5-amide and N-(3'-(3'-deoxydigitoxigenin))-6-carboxyfluorescein-6-amide, where the former is preferred. Additionally, a digitoxin pretreatment solution, a dilution buffer, digitoxin calibrators and digitoxin controls are desirably prepared.

The preferred solutions of these reagents are commercially available from Abbott Laboratories, Abbott Park, Illinois. The tracer formulation presently preferred is 18.2 nanomolar tracer in: 0.1 molar tris buffer at pH 7.5; 0.1% (weight/volume) sodium dodecyl sulfate; 0.1% sodium azide; and 0.01% bovine gamma globulin. The antiserum formulation comprises rabbit serum diluted with TDx® dilution buffer, containing 2% ethylene glycol (volume/volume). The dilution buffer comprises: 0.1 molar sodium phosphate at pH 7.5; 0.1% (weight/volume) sodium azide; and 0.01% (weight/volume) bovine gamma globulin. The pretreatment formulation comprises: 0.1 molar tris buffer at pH 7.5; 0.1% (weight/volume) sodium azide; 0.5% (weight/volume) copper sulfate; and 10.0% (weight/volume) 5-sulfosalicylate. Digitoxin calibrators comprising digitoxin and normal human serum are provided at concentrations of 0.0, 5.0, 10.0, 20.0, 40.0 and 80.0 nanograms per milliliter, with 0.1% sodium azide preservative. Digitoxin controls comprising digitoxin and normal human serum are provided at concentrations of 7.5, 15.0 and 35.0 nanograms per milliliter, with 0.1% sodium azide preservative.

The preferred method of the improved assay of the present invention will now be discussed in detail. Because of the very high binding of digitoxin to serum proteins (>90%), an extraction step is employed which precipitates the proteins in a serum sample while recovering 97% of the digitoxin present. However, the assay is a "homogeneous assay," which means that the end polarization readings are taken from a solution in which bound tracer is not separated from unbound tracer. This is a distinct advantage over radioimmunoassay procedures, for example, where the bound radioactive tracer must be separated from the unbound radioactive tracer before a reading can be taken.

According to the preferred assay procedure of the present invention, digitoxin calibrators, controls and unknown samples must be prepared by the same procedure. In the preferred procedure, which is designed to be used in conjunction with the Abbott TDx® Polarization Analyzer, 100 microliters of the serum or plasma sample are pipetted into a labeled centrifuge tube. A pipette, such as a TDx® Precision Dispenser is filled with methanol, and purged of air bubbles. Three hundred microliters of methanol are then dispensed into each centrifuge tube by touching the end of the dispensing syringe tip to the wall of the centrifuge tube while releasing the methanol. After all samples are pipetted, each centrifuge tube is capped and then mixed on a vortex mixer for three to five seconds. The tubes are then placed into a centrifuge head. The tubes should be evenly distributed so that the centrifuge head is balanced. The samples are then centrifuged for at least three minutes at 9,500 x g, or until clear supernatant and a hard, compact pellet of denatured protein is obtained. After centrifugation is complete, each tube is uncapped and the supernatant is decanted into the corresponding sample well of a TDx® Sample Cartridge or equivalent. Since 300 microliters of sample supernatant are required to perform the assay in accordance with the preferred TDx® assay procedure, care must be taken when decanting to transfer the last drop of supernatant into the sample well. This can be accomplished by tapping the centrifuge tube on the edge of the sample cartridge.

If the TDx® Digitoxin Assay Kit is being used with the TDx® analyzer, the caps from each of the three vials in the TDx® Digitoxin Reagent pack are removed and placed into designated wells inside the TDx® Polarization Analyzer, and the assay procedure from this point is fully automated.

If a manual assay is being performed, then the treated sample is mixed with dilution buffer. The antibody and the pretreatment solution are placed into the test tube containing the sample, and a background reading is taken. Then tracer and dilution buffer are added to the sample, and, after incubation, a fluorescence polarization reading is taken.

The fluorescence polarization value of each calibrator, control or sample is determined and is printed on the output tape of an instrument such as the Abbott TDx® Polarization Analyzer. A standard curve is generated in the instrument by plotting the polarization, P, of each calibrator versus its concentration using a nonlinear regression analysis. The concentration of each control and samples is read off the stored calibration curve and printed on the output tape.

With respect to the foregoing preferred procedure, it should be noted that the digitoxin tracer, antibody, pretreatment solution, calibrators and controls should be used directly after removal from storage at between about 2 and about 8°C, while the dilution buffer should be stored at room temperature. A standard curve and controls should be run every two weeks, with each calibrator and control run in duplicate. Controls should be run with each batch, and all samples can be run in duplicate. It should also be noted that centrifugation must be of sufficient duration (usually at least about three minutes, and sometimes longer) to produce a hard, compact pellet of precipitated protein. Floating pieces of unsedimented protein can cause plugging of the probe and poor reproducibility of sampling. Finally, it should be noted that samples of fresh plasma, samples with abnormally high protein content, or extremely lipemic samples may also fail to yield the desired 300 microliters of supernatant. In such cases, the centrifugation time may be increased, for example, to five minutes, or the volume of the sample may be increased to 150 microliters, or the volume of methanol may be increased to 450 microliters while the three minute centrifugation time is maintained. The sensitivity of the assay is 1.0 nanogram/milliliter of digitoxin, and the crossreactivity of the assay to digoxin is less than 10%. When compared to a commercially available radioimmunoassay, using 178 clinical samples, a linear least-squares regression analysis gave a slope of 1.060, an intercept of 0.729, and a correlation coefficient of 0.967.

It should be understood that the foregoing detailed description and the following Examples are intended to be illustrative, but not limiting, with respect to the scope of the present invention. Various modifications will become apparent to one skilled in the art, and thus it is intended that the scope of the invention be defined by the claims and equivalents thereto.

EXAMPLE 1

Preparation of 3-amino-3-deoxydigitoxigenin

To a warm solution of 0.2 gm of digitoxigenin in 4 ml of acetone was added 0.2 ml of Jones Reagent. The solution was stirred at room temperature for about one-half hour. The solution was diluted with 15 ml of water, and cooled in a freezer. The crystalline ketone was isolated by removal of solvent by pipette. After washing with 15 ml of water, the solid was dissolved in 2 ml of methanol, and 0.34 gm of ammonium acetate and 0.04 gm of sodium cyanoborohydride were added. After stirring at room temperature for about 18 hours, the solution was diluted with 20 ml of water and extracted with three portions of 20 ml of chloroform. The chloroform was removed in vacuo to leave a white foam. The foam was redissolved in a minimum amount of chloroform and diluted with four times the volume of hexane. After one hour, the product, 3-amino-3-deoxydigitoxigenin, was isolated by filtration as a white crystalline material.

EXAMPLE II

Preparation of N-(3'-(3'-deoxydigitoxigenin))-5-carboxyfluorescein-5-amide

A solution of 0.0143 gm of 5-carboxyfluorescein, 0.013 gm of N-hydroxysuccinimide, and 0.0215 gm of 1,3-dicyclohexylcarbodiimide in 0.5 ml of dry pyridine was stirred at room temperature for 18 hours. 0.02 gm of 3-amino-3-deoxydigitoxigenin in 0.2 ml of dry pyridine was added. The solution was stirred at room temperature for 18 hours. Chromatography of the crude reaction solution on 20 x 20 cm 0.5 mm silica gel plates with ethyl acetate/hexane/acetic acid in a ratio of 60/40/2 and collection of the appropriate band with methanol yielded slightly impure product. Rechromatography on two 20 x 20 cm 0.5 mm silica gel plates using diethyl ether/methanol/acetic acid in a ratio of 240/10/1 with multiple elutions produced the 5-carboxyfluorescein conjugate of 3-amino-3-deoxydigitoxigenin.

EXAMPLE III

Preparation of N-(3'-(3'-deoxydigitoxigenin))-6-carboxyfluorescein 6-amide

A solution of 0.01 gm of 6-carboxyfluorescein, 0.009 gm of N-hydroxysuccinimide, and 0.015 gm of dicyclohexylcarbodimide in 1/2 ml of dry pyridine was stirred at room temperature for two hours. A solution of 0.014 gm of 3-amino-3-deoxydigitoxigenin in 0.2 ml of dry pyridine was added. After stirring at room temperature for 18 hours, the crude reaction was placed on a 20 x 20 cm 2.0 mm silica gel plate and eluted with hexane/ethyl acetate/ acetic acid in a ratio of 20/30/1. Collection of the appropriate band with methanol yielded pure 6-carboxyfluorescein 3-amino-3-deoxydigitoxigenin conjugate.

**Claims**

1. A reagent for quantitively determining the presence of digitoxin in blood serum in a homogeneous mixture of said reagent, where said reagent comprises the reaction product of 3-amino-3-deoxydigitoxigenin and one of the group consisting of 5-carboxyfluorescein and 6-carboxyfluorescein.

2. The reagent of Claim 2, wherein the reagent comprises N-(3'-(3'-deoxydigitoxigenin))-5-carboxyfluorescein-5-amide.

3. A process for measuring concentration of digitoxin which comprises the steps of:

contacting a sample with a digitoxin antiserum;

contacting the resulting solution with a carboxyfluorescein digitoxigenin derivative capable of producing a detectable fluorescence polarization response to the presence of the digitoxin antiserum and to form a homogeneous test solution;

passing plane polarized light through the homogeneous test solution to obtain a fluorescence polarization response; and

detecting the fluorescence polarization response of the homogeneous test solution to measure the amount of digitoxin in the sample.

4. The process of Claim 3, wherein the carboxyfluorescein digitoxigenin derivative is N-(3'-(3'-deoxydigitoxigenin))-5-carboxyfluorescein-5-amide.

5. The process of Claim 3, wherein the carboxyfluorescein digitoxigenin derivative is N-(3'-(3'-deoxydigitoxigenin))-6-carboxyfluorescein-6-amide.

6. A method for making N-(3'-(3'-deoxydigitoxigenin-5-carboxyfluorescein-5-amide or N-(3'-(3'-deoxydigitoxigenin))-6-carboxyfluorescein-6-amide comprising the step of reacting 5-carboxyfluorescein or 6-carboxyfluorescein with 3-amino-3-deoxydigitoxigenin under conditions suitable for forming amide linkages.

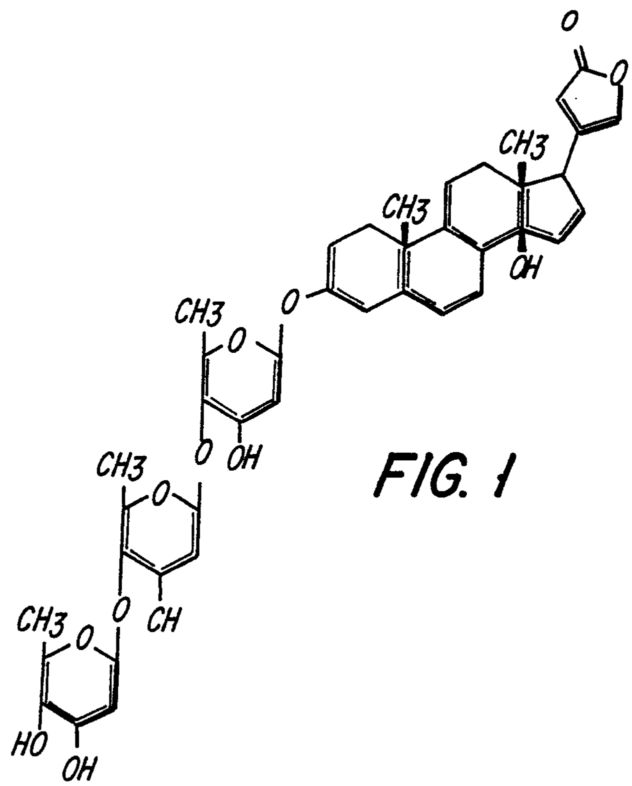

FIG. 1

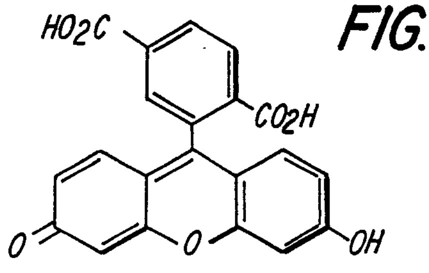

FIG. 5

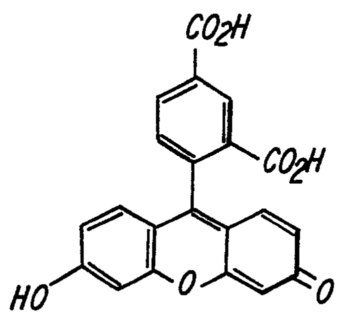

FIG. 2

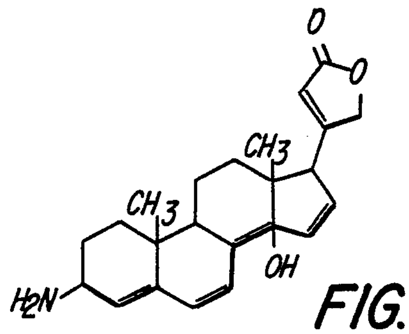

FIG.3

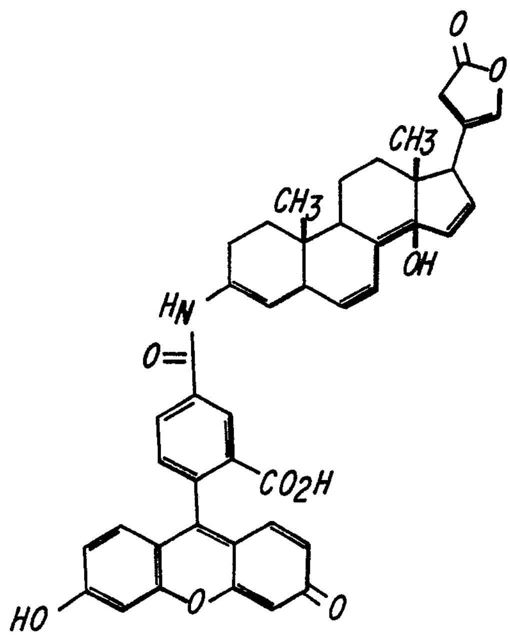

FIG. 4

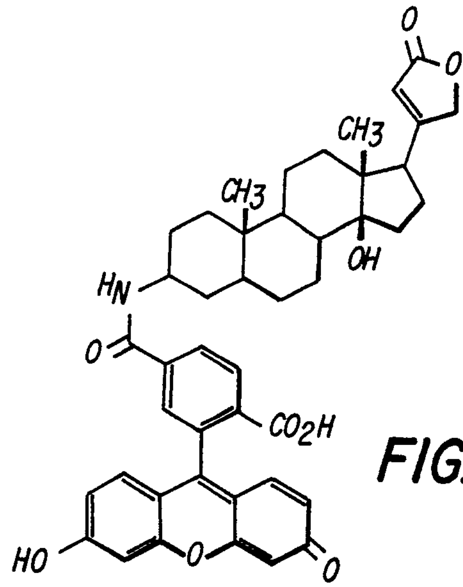

FIG.6

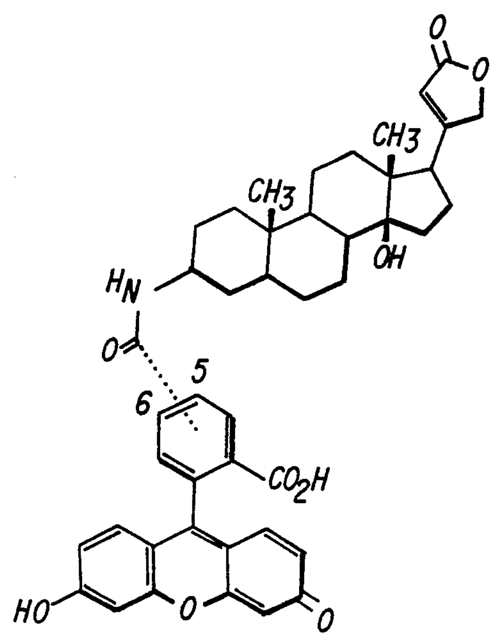

FIG. 7

FIG. 8a

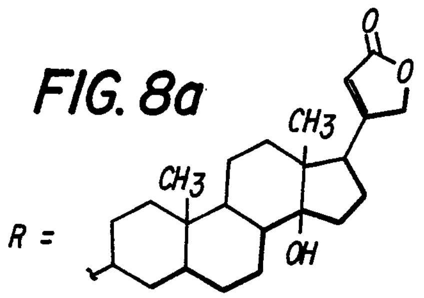

FIG.8b

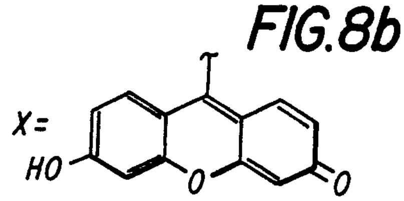

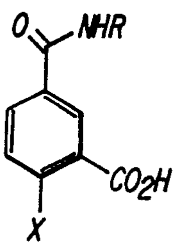

FIG. 8(I)

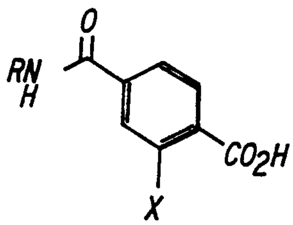

FIG.8(II)

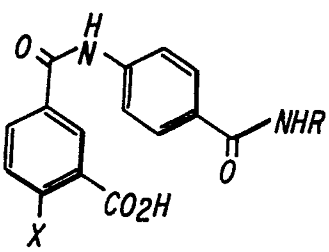

FIG.8(III)

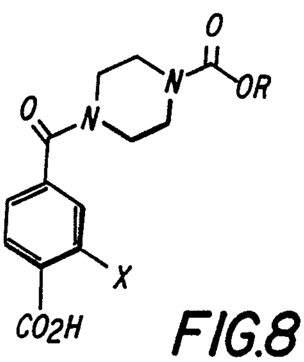

FIG.8(IV)

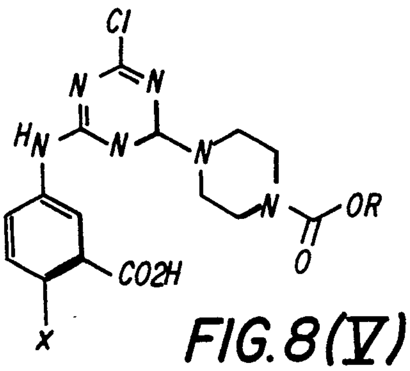

FIG. 8(V)

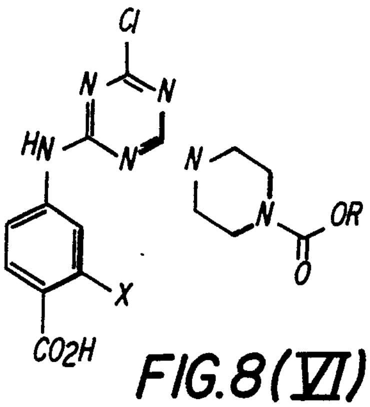

FIG. 8(VI)

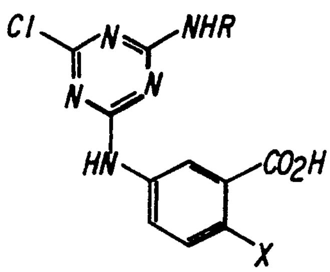

FIG. 8(VII)

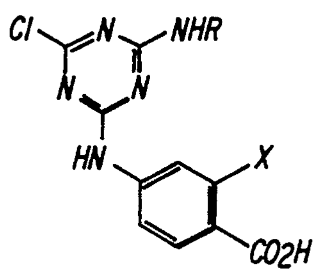

FIG. 8(VIII)

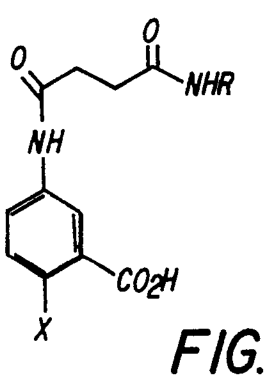

FIG. 8(IX)

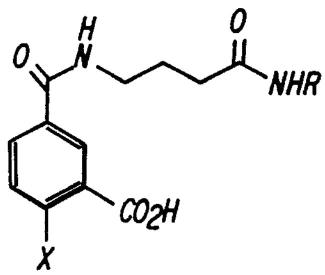

FIG. 8(X)

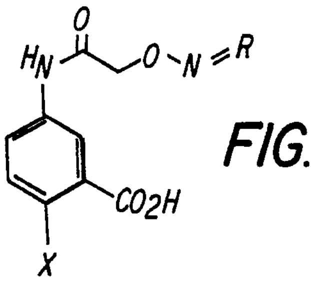

FIG.8(XI)

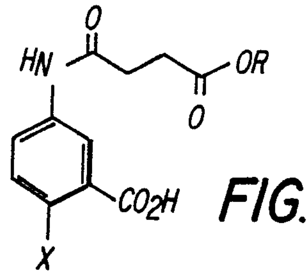

FIG.8(XII)

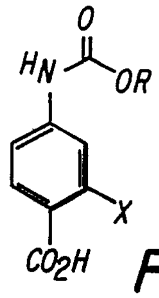

FIG.8(XIII)

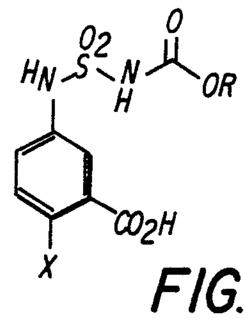

FIG.8(XIV)

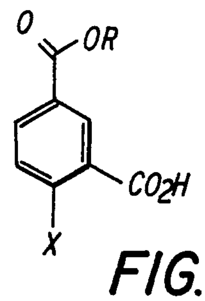

FIG.8(XV)

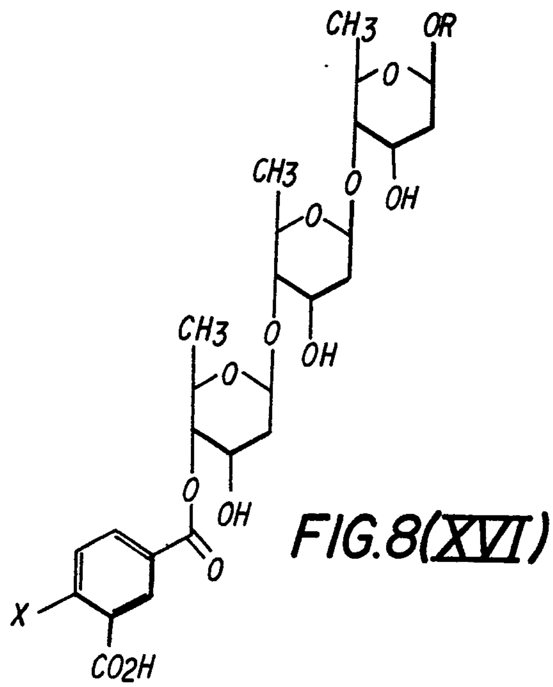

FIG.8(XVI)